# EUROPÄISCHE PATENTSCHRIFT

(11) **EP 2 522 387 B1**
(45) Veröffentlichungstag und Bekanntmachung des Hinweises auf die Patenterteilung: **31.05.2017**
(21) Anmeldenummer: 12164371.2
(22) Anmeldetag: 17.04.2012
(51) Int. Cl.: A61N 1/05, A61N 1/08

(54) **Implantierbare medizinische Leitung**
Implantable medical lead
Ligne médicale implantable

(30) Priorität: 10.05.2011 US 201161484239 P
(43) Veröffentlichungstag der Anmeldung: 14.11.2012
(73) Patentinhaber: BIOTRONIK SE & Co. KG, 12359 Berlin (DE)
(72) Erfinder: Bartels, Klaus, 10115 Berlin (DE); Kolberg, Gernot, 12161 Berlin (DE); Palm, Jochen, 15831 Mahlow (DE)
(74) Vertreter: Galander, Marcus

(56) Entgegenhaltungen:
- EP-A2- 1 872 825
- EP-A2- 2 465 573
- US-A1- 2008 154 348
- US-A1- 2010 217 262

## Beschreibung

Die Erfindung betrifft eine implantierbare medizinische Leitung zur Übertragung elektrischer Impulse an reizbares Körpergewebe und/oder von an Körpergewebe erfassten Signalen zu einer Erfassungs- und Auswertungseinheit, mit einer distalen Elektrode oder einem distalen Sensor bzw. Aktuator, einem proximalen Elektroden- oder Sensor-/Aktuatoranschluss und einem die Elektrode oder den Sensor/Aktuator mit dem Elektroden- oder Sensor/Anschluss verbindenden, in einem Leitungskörper verlaufenden Leitungspol. Dies sind insbesondere Elektrodenleitungen, es kann sich aber auch um Anschlussleitungen von intrakorporal eingesetzter Sensorik bzw. Messtechnik handeln.

Medizinische Implantate wie Schrittmacher und Defibrillatoren besitzen häufig eine elektrische Verbindung zum Körperinneren des Patienten. Eine solche Verbindung dient zur Messung von elektrischen Signalen und/oder zur Stimulation von Körperzellen. Diese Verbindung stellt in der Regel eine Elektrodenleitung der o. g. Art dar. Gegenwärtig werden elektrische Signale zwischen dem Implantat und den Elektrodenkontakten (Spitze, Ringe, HV-Schockwendeln, Sensoren o. ä.) mit elektrisch gut leitenden Materialien übertragen.

Wird ein System aus Implantat und Elektrode starken Störfeldern (EMI, MRI) ausgesetzt, so kann es zu unerwünschtem Fehlverhalten kommen, speziell einer Erwärmung von Teilen des Systems oder elektrischen Fehlfunktionen (z. B. Resets). Die Erwärmung kann zu Schädigungen von Körpergewebe oder von Organen führen, wenn die erwärmten Teile direkten Kontakt zum Gewebe haben. Dies ist insbesondere bei der Elektrodenspitze der Fall.

Die Ursache für das unerwünschte Fehlverhalten ist die Wechselwirkung des Feldes mit der länglichen Leitungsstruktur der Elektrode: Die Elektrode wirkt als eine Antenne und empfängt Energie aus den umgebenden Feldern. Diese Energie auf den therapeutisch genutzten Leitungen kann die Antenne distal über die Elektrodenkontakte (Spitze, Ring ...) an das Gewebe oder proximal an das Implantat abgeben. Die ähnlichen Probleme treten auch bei anderen länglichen leitfähigen Strukturen auf, deren proximales Ende nicht zwingend mit einem Implantat verbunden ist (z. B. bei Kathetern, temporären Elektroden etc.). Bekannt sind abgeschirmte Elektroden. Die Schirmung der Elektrode wirkt vor allem gegen von außen einkoppelnde elektrische Felder. Außerdem sind diese Schirmungen nur mit einer entsprechenden Schirmstärke wirksam und langzeitstabil. Es ist daher ein Kompromiss zu finden zwischen einer Vergrößerung des Elektrodendurchmessers - mit entsprechenden Auswirkungen für die Kosten und die Handlichkeit der Elektrode - und Einbußen in der Schirmwirkung. Wegen der hohen Anforderungen an die Biokompatibilität bzw. Biostabilität, können Materialien, die sich bzgl. ihrer abschirmenden Wirkung bewährt haben, z. B. weichmagnetische Nickel-Eisen-Legierungen, nicht eingesetzt werden.

Zur Vermeidung von Störungen durch magnetische Wechselfelder und insbesondere in Magnetresonanz-Apparaturen (MRI), speziell zur Begrenzung von Erwärmungen der Elektrodenspitze in solchen Feldern, wurde in der US 2008/0243218 A1 das Vorsehen eines in Längsrichtung abwechselnd hin und her geführten Schutz-Leiters in einer Elektrodenleitung vorgeschlagen. Dieses sogenannte Billabong-Prinzip nutzt Gegeninduktivitäten zur Minderung induzierter Ströme. Allerdings ist in diesem Fall durch die dreilagige Wendelwicklung ebenfalls eine Vergrößerung des Elektrodendurchmessers zu erwarten.Relevanter Stand der Technik wird z.B. in den Dokumenten US 2010/0217262 A1, EP1872825 A2 und EP2465573 A2 offenbart.

Eine implantierbare medizinische Leitung nach dem Oberbegriff von Anspruch 1 ist aus der US 2008/0154348 A1 bekannt. Der Erfindung liegt die Aufgabe zugrunde, eine verbesserte implantierbare Leitung der genannten Art bereit zu stellen, die in starken äußeren Wechselmagnetfeldern verbesserte Eigenschaften aufweist und konstruktiv leicht und somit kostengünstig realisierbar ist. Diese Aufgabe wird durch eine implantierbare Leitung mit den Merkmalen des Anspruchs 1 gelöst. Zweckmäßige Fortbildungen des Erfindungsgedankens sind Gegenstand der abhängigen Ansprüche.

Beispiele, Aspekte und Methoden, die nicht unter den Schutzumfang der Ansprüche fallen sind nicht Teil der Erfindung und werden hier zum besseren Verständnis und beispielhaft dargestellt. Die Erfindung schließt den Gedanken ein, zur Verwirklichung des erwähnten Billabong-Prinzips mehrere separate und gegen einander isolierte Leiter (speziell solche einer an sich vorliegenden Leitungsstruktur) zu nutzen, statt einen zusammenhängenden Leiter zu "falten". Sie schließt weiter den Gedanken ein, die separaten Leiter an einer Stelle, welche dann als Umkehrpunkt im Leitungsverlauf wirkt, miteinander elektrisch zu verbinden. Es versteht sich, dass zur Vermeidung irregulärer Signalübertragungen dann mindestens einer der separaten Leiter an mindestens einem seiner Enden nicht angeschlossen sein darf.

Die bei Umsetzung der Billabong-Schaltung verbleibenden Adern haben auch ohne ohmschen Kontakt einen dämpfenden Effekt oder sie sind im Knoten noch kontaktiert, laufen parallel mit offenem Ende und wirken durch Fehlanpassung als Energiesenke (Lercherleitung).

Ohne Ausnutzung des Billabong-Prinzips ergeben sich noch folgende Varianten:
1. Dem durchlaufenden therapeutischen Leiter werden schrittweise mit axialem Abstand weitere Leiter parallel geschaltet, die an ihrem Ende offen sind und den Wellenwiderstand der Leitung axial absenken.
2. Parallel zum therapeutischen Leiter sind weitere Leiter parallel geschaltet, die zum Elektrodenkopf hin abgeschaltet werden. So wird der Wellenwiderstand in Richtung Kopf erhöht. Die eingekoppelte Energie wird reflektiert.

In zweckmäßigen und weitgehend durch die technologischen Gegebenheiten bei der Herstellung verschiedenartiger medizinischer Leitungen geprägten Ausführungen sind die separaten Leiter durch eine Löt-, Schweiß- oder Klebverbindung mittels leitfähigen Klebstoffs fest miteinander verbunden.

In einer weiteren, auch unter technologischen Gesichtspunkten neuartigen Ausführung sind die separaten Leiter durch Crimpen, insbesondere mittels Crimpschelle, fest miteinander verbunden. Die letztgenannte Verbindungsmethode ist, bei geeigneter Ausgestaltung, technologisch besonders einfach und somit kostengünstig. Der Ort der Verbindungsstelle kann gerade mit einer Crimpverbindung besonders flexibel gewählt werden, obwohl ein konstanter Crimpprozess eingesetzt wird. So lassen sich Zuleitungen herstellen, die individuell an die herrschenden Bedingungen angepasst und so bzgl. ihrer Erwärmung optimiert werden können.

Die so gestalteten Verbindungsstellen werden anschließend in geeigneter Form mit einem isolierenden Material geschützt. Sie können verklebt, eingegossen, extrudiert oder ummantelt werden.

Zur Verknüpfung einer zusätzlichen Funktionalität mit der Ausführung der Erfindung ist in weiteren Ausführungsformen im Übergabepunkt bzw. Umkehrpunkt ein oder mehrere elektrische Bauelemente, insbesondere ein Widerstand, eine Kapazität, eine Induktivität, ein Filterbauelement, ein integrierter Schaltkreis, ein Sensorbauelement, oder ein Aktuator vorgesehen. Als Beispiel für einen Aktuator sei eine implantierbare Medikamentenpumpe genannt, die über die erfindungsgemäße Zuleitung angesteuert wird. Ein anderes Beispiel sind mechanische Einrichtungen am Elektrodenende zur Fixierung der Elektrode, die über das Kabel bedient werden. Hiermit lassen sich etwa die grundlegenden elektrischen Eigenschaften der Leitung auf bestimmte Einsatzbedingungen hin optimieren, oder die Leitung kann den Funktionsumfang oder die Funktionsfähigkeit eines daran angeschlossenen Medizin-elektronischen Geräts verbessern.

In konstruktiven Varianten der Erfindung ist vorgesehen, dass der Leitungspol n≥2 separate und gegeneinander isolierte Leiter umfasst, von denen jeweils zwei oder mehr derart an je einem Übergabe- oder Umkehrpunkt elektrisch miteinander verbunden sind, dass der Leitungspol einen bis (n -1) Umkehrpunkte aufweist. Dies kann speziell so ausgestaltet sein, dass der oder ein Elektrodenpol 4 entlang der Achse der Leitung parallel verlaufende oder wendelförmig um die Achse verlaufende separate und gegeneinander isolierte Leiter aufweist, wobei ein oder zwei Umkehrpunkte gebildet sind.

Die Erfindung lässt sich ohne Weiteres bei den weitverbreiteten multipolaren Elektrodenleitungen einsetzen, dergestalt, dass sie mehrere Leitungspole umfasst, die jeweils mindestens zwei separate und gegeneinander isolierte Leiter und mindestens einen Übergabe- bzw. Umkehrpunkt aufweisen.

Eine an weit verbreitete Elektrodenleitungstypen angepasste Ausführung sieht vor, dass die separaten Leiter des oder eines Leitungspols parallel zueinander verlaufende gestreckte, insbesondere seilartig geflochtene, Leiter sind. Eine an einen weiteren verbreiteten Typ von Elektrodenleitungen angepasste Ausführung zeichnet sich dadurch aus, dass die separaten Leiter des oder eines Leitungspols als, insbesondere ineinander gewickelte oder koaxial zu einer Längsachse der Leitung angeordnete, Leiterwendeln ausgebildet sind. In einer noch weiteren Ausführungsform liegt jeweils einer der separaten Leiter des oder eines Elektrodenpols auf einem von Elektrodenpotentialen unabhängigen Potential und hat insbesondere eine Abschirmwirkung.

Neben Vorrichtungsaspekten hat die Erfindung auch herstellungstechnische Aspekte, die in einem Verfahren mit den Merkmalen des Anspruchs 11 zum Ausdruck kommen. Insoweit zeichnet sich die Erfindung dadurch aus, dass anfangs bereitgestellte separate und gegeneinander isolierte Leiter lokal gezielt abisoliert und an den abisolierten Stellen zur Bildung von Übergabe- oder Umkehrpunkten miteinander elektrisch verbunden werden und die so ausgebildeten Leitungspole erst anschließend an den Leitungskörper eingebettet werden.

In einer verfahrensseitigen Ausgestaltung ist vorgesehen, dass das Zerstören der Isolierung und elektrische Verbinden der separaten Leiter zur Bildung des Übergabe- oder Umkehrpunktes durch Laserbearbeitung oder Widerstandsschweißen erfolgt. Eine hierzu alternative, technologisch besonders einfache und kostengünstige Ausgestaltung sieht vor, dass das Zerstören der Isolierung und elektrische Verbinden der Leiter zur Bildung des Umkehrpunktes durch Crimpen, insbesondere unter Einsatz einer Crimpschelle, erfolgt.

Unter dem Aspekt technologischer Einfachheit und der Nutzung etablierter Verfahren der Kunststoffverarbeitung und somit im Hinblick auf niedrige Kosten ist eine weitere Ausgestaltung vorteilhaft, bei der die Bildung des Leitungskörpers durch Umspritzen des Elektrodenpols, insbesondere zusammenhängendes Umspritzen mehrerer Elektrodenpole, oder Extrudieren eines Kunststoffkörpers um den Elektrodenpol oder die Elektrodenpole ausgeführt wird.

Weitere Beispiele werden wie folgt beschrieben:
1. Die Umkehrpunkte sind als elektrische Kurzschlusspunkte ausgeführt.
2. Je Elektrodenpol sind mindestens 4 entlang der Symmetrieachse des Leadbodys voneinander elektrisch isolierte Leiter angeordnet, die in ihrer Querschnittsfläche x-bzw. sternförmig angeordnet sind, die entlang des Leadbodys mindestens jeweils 1 Übergabe- oder Umkehrpunkt besitzen.
3. Je Elektrodenpol sind mindestens 4 wendelförmig um einen Kern entlang der Symmetrieachse des Leadbodys ausgebildete, voneinander elektrisch isolierte Leiter angeordnet, die in ihrer Querschnittsfläche x- bzw. sternförmig angeordnet sind, die entlang des Leadbodys jeweils mindestens 1 Umkehrpunkt besitzen und dass jeweils 2 oder mehrere Leiter elektrisch parallel geschaltet sind.
4. Je Elektrodenpol sind mindestens 4 wendelförmig um einen Kern entlang der Symmetrieachse des Leadbodys ausgebildete, voneinander elektrisch isolierte Leiter angeordnet, die in ihrer Querschnittsfläche x- bzw. sternförmig angeordnet sind, die entlang des Leadbodys jeweils mindestens 1 Übergabe- oder Umkehrpunkt besitzen und dass jeweils einer der elektrisch isolierten Leiter auf einem von den Elektrodenpotentialen unabhängigen Potential liegt (Abschirmung).
5. Die 2 bis 9 Leiter werden in einem Komplex voneinander isoliert zusammengefasst.
6. Die Leiter in einem Komplex sind verdrillt.
7. Zwischen mindestens zwei Leitern in einem Komplex existiert mindestens ein Übergabe- oder Umkehrpunkt.
8. Zwischen mindestens zwei Leitern in einem Komplex existiert mindestens ein Positionswechsel.
9. Im Umkehrpunkt oder dem Positionswechsel sitzt ein elektrischer Widerstand.
10. Im Umkehrpunkt oder dem Positionswechsel sitzt eine elektrische Kapazität.
11. Im Umkehrpunkt oder dem Positionswechsel sitzt eine elektrische Induktivität.
12. Im Umkehrpunkt oder dem Positionswechsel sitzen ein oder mehrere elektromechanische Elemente (Filter).
13. Im Umkehrpunkt oder dem Positionswechsel sitzen ein oder mehrere elektrische Schaltkreise.
14. Im Umkehrpunkt oder dem Positionswechsel sitzt eine Kombination aus den oben genannten elektrischen Elementen.
15. Im Umkehrpunkt oder dem Positionswechselsitzen ein oder mehrere Sensoren.
16. Mindestens 2 der Leiter in einem Komplex werden partiell durch einen Laser freigelegt.
17. Mindestens einer der Leiter in einem Komplex wird mit einem Laser durchtrennt.
18. Mindestens zwei der Leiter in einem Komplex werden mit einem Laser zusammen geschweißt.
19. Mindestens zwei der Leiter in einem Komplex werden mit einem Widerstandsschweißgerät zusammen geschweißt.
20. Mindestens zwei der Leiter in einem Komplex werden zusammen gelötet.
21. Mindestens 2 der Leiter in einem Komplex werden durch eine Crimpschelle miteinander verbunden.
22. Umkehr- und Umleitungspunkte bilden ein Leitungsprinzip ab.
23. Die Leiter in einem Komplex sind verdrillt.
24. Der Leiterkomplex wird im Leitungskörper parallel zur Achse geführt.
25. Der Leiterkomplex wird im Leitungskörper um die Achse gewendelt geführt.
26. Der Leiterkomplex verläuft im Leitungskörper gewendelt mit variierender Steigung (inklusive parallelem Verlauf).
27. Der Leiterkomplex verläuft im Leitungskörper gewendelt mit einer Steigungsumkehr der Wendel.
28. In dem Kern kann ein weiterer isolierter Leiter verlaufen. Dieser Leiter kann mit einem Lumen ausgestattet sein. Er kann auch dreh- oder verschiebbar zur Übertragung von Kräften oder Momenten ausgestaltet sein. Er kann auch als Lichtleiter ausgestaltet sein.

Vorteile und Zweckmäßigkeiten der Erfindung ergeben sich im Übrigen aus den nachfolgenden Beschreibungsbeispielen anhand der Figuren. Von diesen zeigen:
- Fig. 1: eine schematische Darstellung einer herkömmlichen implantierbaren Elektrodenleitung,
- Fig. 2: in einer perspektivischen Schnittsdarstellung ein Beispiel einer hochentwickelten Elektrodenleitung mit mehreren in einem Leitungskörper aufgenommenen Zuleitungen,
- Fig. 3: in einer perspektivischen Schnittdarstellung eine weitere hochentwickelte Elektrodenleitung mit mehreren Zuleitungen in koaxialer Anordnung,
- Fig. 4A und 4C: schematische Darstellungen von Ausführungsbeispielen der Erfindung, als teilweise geschnittene perspektivische Darstellung,
- Fig. 5: eine perspektivische Darstellung eines weiteren Ausführungsbeispiels der Erfindung,
- Fig. 6: eine perspektivische Darstellung einer Abwandlung der in Fig. 4A gezeigten Ausführung,
- Fig. 7: eine perspektivische Darstellung zur Verdeutlichung der Anordnung des in Fig. 6 gezeigten Leiterkomplexes in einem Leitungskörper,
- Fig. 8: eine schematische Darstellung einer vierpoligen Elektrodenleitung in einer ersten erfindungsgemäßen Realisierung,
- Fig. 9: eine schematische Darstellung einer vierpoligen Elektrodenleitung in einer zweiten erfindungsgemäßen Realisierung,
- Fig. 10: eine schematische Darstellung einer vierpoligen Elektrodenleitung in einer dritten erfindungsgemäßen Realisierung,
- Fig. 11A und 11B: schematische Darstellungen von Verbindungsvarianten, die erfindungsgemäß in einer zweipoligen Zuleitung realisiert werden können,
- Fig. 12A und 12B: schematische Darstellungen von Verbindungsvarianten, die erfindungsgemäß in einer dreipoligen Zuleitung realisiert werden können, sowie
- Fig. 13: eine schematische Darstellung einer Verbindungsvariante, die erfindungsgemäß in einer vierpoligen Zuleitung realisiert werden kann.

In der nachfolgenden Beschreibung der Figuren sind jeweils für gleiche oder gleich wirkende Teile oder Abschnitte ähnliche Bezugsziffern verwendet, und vorangegangene Beschreibungen werden, soweit sie sich auf solche Teile beziehen und keine Besonderheiten gegeben sind, bei nachfolgenden Figuren nicht wiederholt.

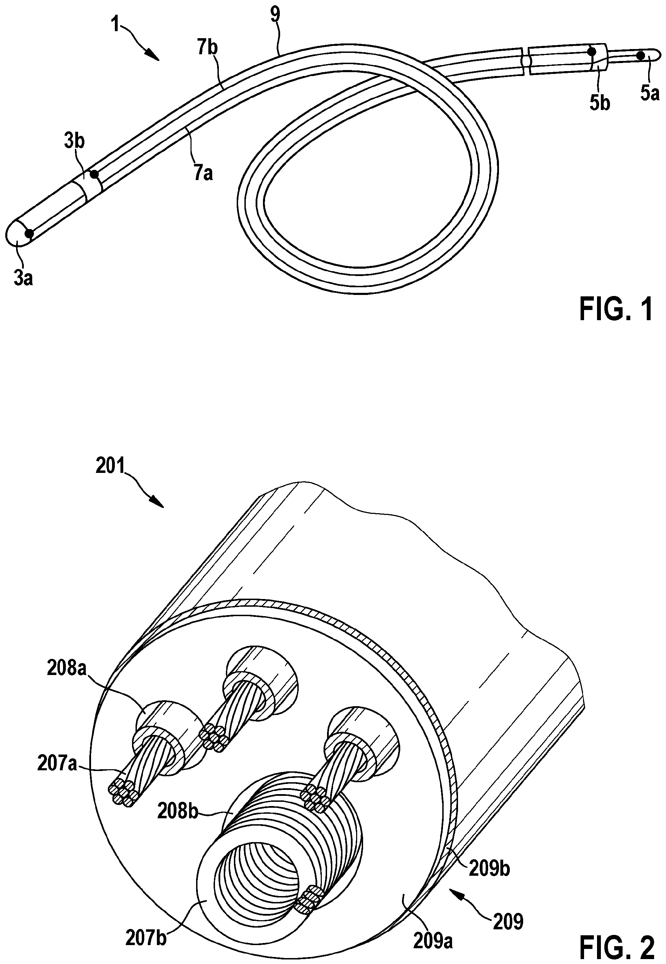

Fig. 1 zeigt schematisch eine bipolare Elektrodenleitung 1, an deren distalen Ende eine Spitzenelektrode 3a und eine Ringelektrode 3b und an deren proximalen Ende zwei entsprechende Elektrodenkontakte 5a und 5b vorgesehen sind, die mit der jeweils zugehörigen Elektrode durch eine erste und zweite Zuleitung 7a, 7b verbunden sind. Die Elektroden, Elektrodenkontakte und Zuleitungen sind auf bzw. in einem (in der Regel mehrschichtigen) Leitungskörper 9 untergebracht.

Fig. 2 zeigt in einer perspektivischen Schnittansicht mit verschiedenen Schnittebenen eine moderne Elektrodenleitung 201, bei der in einem Innenschlauch 209a - als Kern eines Zuleitungskörpers 209 - drei Lumen 208a mit kleinerem Durchmesser und ein weiteres Lumen 208b mit größerem Durchmesser vorgesehen sind. In jedem der kleineren Lumen 208a befindet sich eine Elektrodenzuleitung 207a mit Seilstruktur, die ihrerseits mit einem (nicht gesondert bezeichneten) isolierendem Mantel, z. B. aus PTFE, ETFE oder PI, versehen ist. Im größeren Lumen 208b verläuft eine Zuleitungswendel 207b, die während der Implantation zur Versteifung der Elektrodenleitung einen Führungsdraht aufnehmen kann. Zur Verbesserung der Gleit- und Abriebeigenschaften des Leitungskörpers 209 hat dieser eine Außenhülle 209b, die diese Eigenschaften positiv beeinflusst.

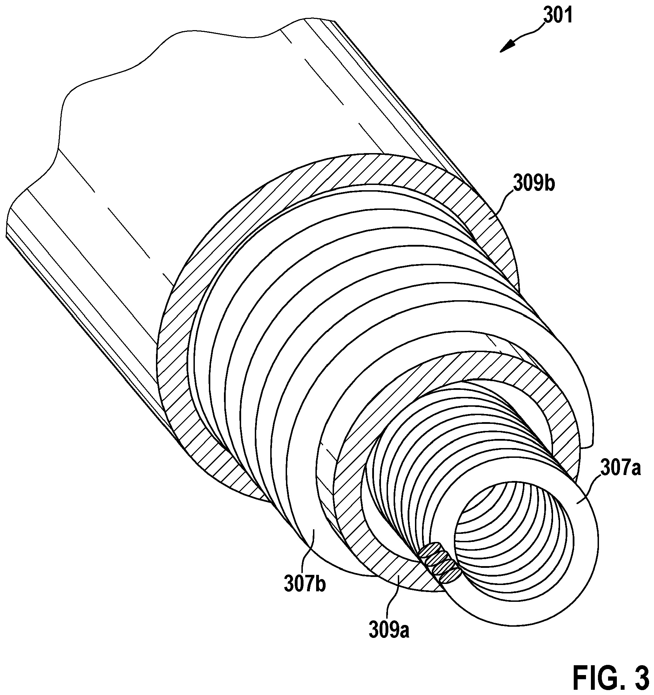

Fig. 3 zeigt eine weitere Ausführung einer implantierbaren Elektrodenleitung, bei der in koaxialer Anordnung als erste Elektrodenzuleitung (bzw. erste Gruppe von Zuleitungen) eine aus mehreren Einzeldrähten gewickelte Innenwendel 307a und koaxial hierzu eine gleichfalls aus mehreren Einzeldrähten gewickelte Außenwendel 307b angeordnet ist (die ebenfalls eine Gruppe von Elektrodenzuleitungen realisieren kann). Zwischen der Innen- und Außenwendel ist ein Silikonschlauch 309a vorgesehen, und die Außenwendel ist mit einem weiteren Isolierschlauch 309b ummantelt, der ebenfalls aus Silikon oder einem Polyurethan oder einem Copolymer bestehen kann. Auch eine Kombination aus mehreren Schläuchen ist hier einsetzbar.

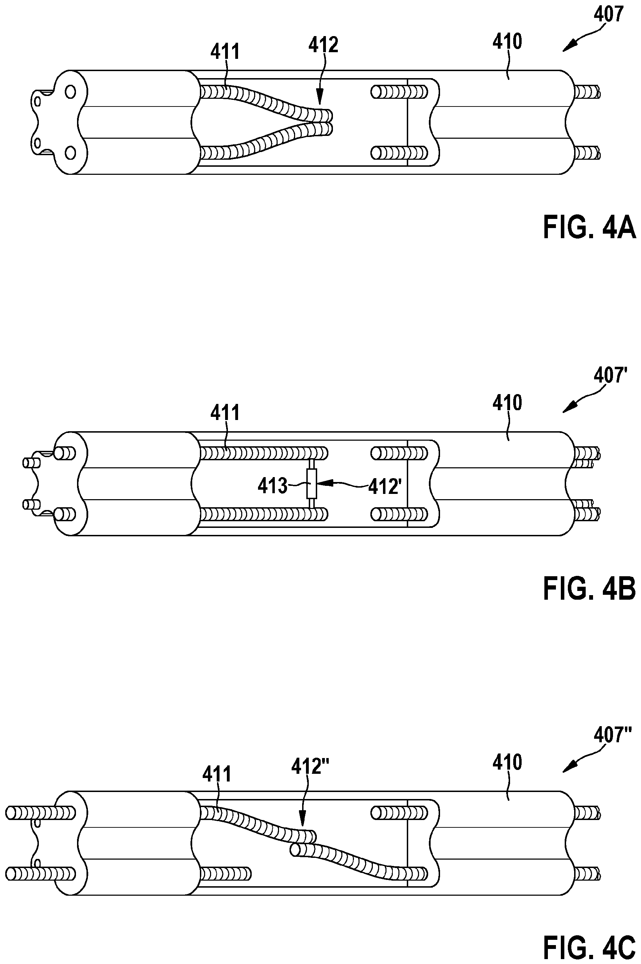

Fig. 4A zeigt eine Zuleitung 407, die in einen (nicht gezeigtem) Leitungskörper eingebettet und dann Bestandteil einer Elektrodenleitung sein kann. Die Zuleitung 407 ist vierpolig ausgebildet, umfasst also vier separate, in einen Kunststoff-Zuleitungskörper 410 eingebettete, seilartig geflochtene Einzelleiter 411. In der Figur ist zu erkennen, dass zwei der Einzelleiter 411 in ihrem Leitungsverlauf durchtrennt und zwei der dadurch frei gewordenen Enden an einem Verbindungspunkt 412, etwa durch Verschweißen, Löten oder leitfähiges Verkleben, leitend miteinander verbunden sind.

Wenn von einem Ende der Zuleitung 407 in den einen der beiden miteinander verbundenen Leiter 411 ein elektrisches Signal eingespeist wird und am anderen der beiden miteinander verbundenen Leiter ein entsprechendes Signal abgegriffen wird, wirkt der Verbindungspunkt 412 als Umkehrpunkt eines durch die elektrische Verbindung beider Leiter geschaffenen zusammenhängenden Leitungsverlaufs. Mit diesem Umkehrpunkt lässt sich eine dem Billabong-Prinzip entsprechende, auf der Gegeninduktivität von in Gegenrichtung mit einem Strom durchschlossenen Leiterabschnitten beruhende Wirkung erreichen, mit der der Einfluss externer Magnetfelder auf die Elektrodenleitungsstruktur reduziert werden kann.

Fig. 4B zeigt eine weitere Zuleitung 407', die eine abgewandelte Ausführung der in Fig. 4A gezeigte und oben beschriebene Zuleitung darstellt. Auch hier sind mindestens zwei der im Zuleitungskörper 410 verlaufenden Einzelleiter 411 in ihrem jeweiligen Leitungsverlauf durchtrennt und in einem Verbindungspunkt (Umkehrpunkt) 412' miteinander elektrisch verbunden. In diese elektrische Verbindung ist hier jedoch ein elektrisches oder elektronisches Bauelement 413 eingebunden, bei dem es sich bspw. um einen ohmschen Widerstand, eine Induktivität, eine Kapazität oder ein Filterelement handeln kann. Auch das Vorsehen eines Sensorbauelements im Umkehrpunkt ist möglich und kann im Hinblick auf spezielle Funktionen von über die Elektrodenleitung angeschlossenen Geräten vorteilhaft sein.

Fig. 4C zeigt als weitere Variante eine Zuleitung 407", bei der wiederum zwei Einzelleiter 411 in ihrem jeweiligen Leitungsverlauf durchtrennt und in einem Verbindungspunkt 412" miteinander elektrisch verbunden sind. Hier stellt der Verbindungspunkt jedoch keinen Umkehrpunkt dar, sondern hat einen Positionswechsel im Leitungsverlauf (Leitungsanfang verglichen mit Leitungsende) zur Folge.

Der Positionswechsel verändert den Abstand zwischen den Leitern und stellt damit die Wellenwiderstände ein, aus denen sich Fehlanpassungen ergeben, die die Energie in der Leitung umsetzen, anstatt sie zur Spitze zu übertragen. Dieser Abstand lässt sich nach dem Konzept der Erfindung konstruktiv sehr leicht einstellen und sogar variieren, indem in einem Leiterbündel Leiter verschiedener Abstände wählbar sind. Er bestimmt das Maß der Kopplung der parallel geführten Leiter und damit den Effekt der Dämpfung. So besteht die Möglichkeit, die Elektrode auf verschiedene Anforderungen, wie z. B. 1,5-Tesla- oder 3-Tesla-Geräte, einzustellen.

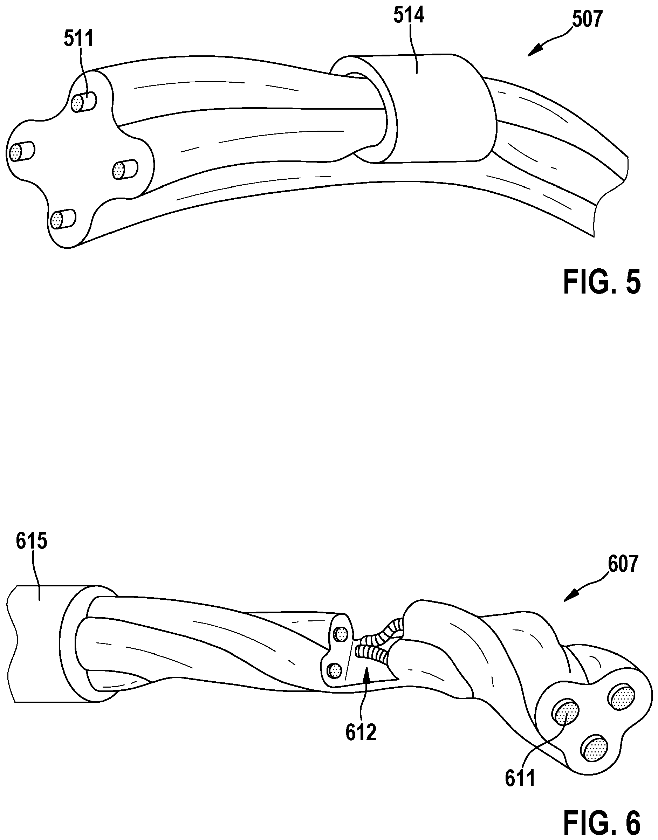

Fig. 5 zeigt am Beispiel einer Zuleitung 507 der in Fig. 4A und 4B gezeigten Art skizzenhaft eine mögliche Realisierung der gleichzeitigen Unterbrechung des Leitungsverlaufes zweier Einzelleiter 511 und ihrer gegenseitigen Verbindung an einem Umkehrpunkt. Hierzu dient eine Crimpschelle 510, die über einen Teil des Umfangs der Zuleitung 507 derart gepresst wird, dass die Leitungsunterbrechung und wechselseitige elektrische Verbindung der benachbarten Leiter 511 durch die mechanische Einwirkung der an ihrem Innenumfang geometrisch geeignet konfigurierten Schelle 514 bewirkt wird.

Fig. 6 zeigt als weitere Modifikation der in Fig. 4A dargestellten Ausführung der Erfindung eine dreipolige Zuleitung 607 mit drei Einzelleitern 611, von denen zwei an einem Verbindungs- und Umkehrpunkt 612 miteinander verbunden sind.

Eine isolierende Verkapselung des Umkehrpunktes 612 wie auch der ihm gegenüber liegenden "blinden" Enden der durchtrennten benachbarten Leiter 611 erfolgt durch eine aufextrudierte Isolierhülle 615, die im linken Teil der Figur lediglich partiell dargestellt ist, sich bei der fertigen Zuleitung aber über deren gesamte Länge erstreckt.

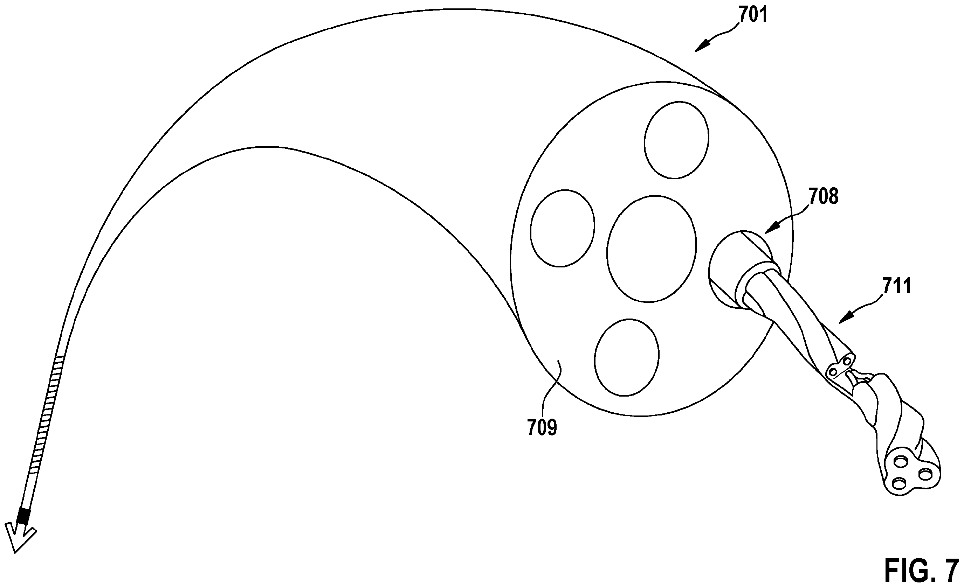

Fig. 7 zeigt, wie die durch den aufextrudierten Schlauch 615 geschützte und fertig gestellte Zuleitung 711 in einem der kleineren Lumen 708 eines Elektrodenleitungskörpers 709 einer Elektrodenleitung 701 untergebracht wird.

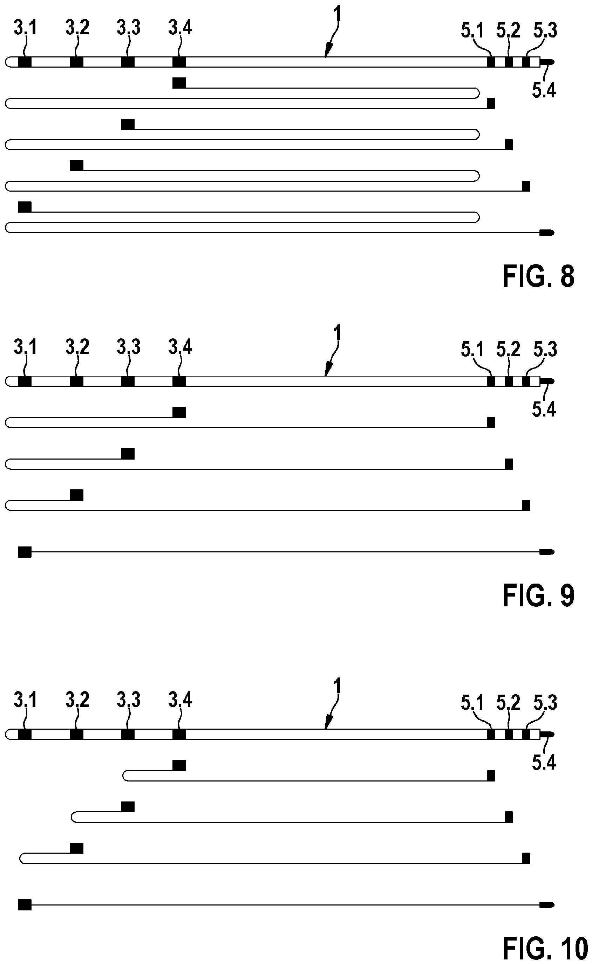

In den Figuren 8 bis 10 sind verschiedene Varianten der Verbindung von Elektrodenkontakten 5.1 bis 5.4 einer vierpoligen Elektrodenleitung 1 mit ihren im distalen Bereich angeordneten Elektroden 3.1 bis 3.4 schematisch dargestellt. Bei der Variante nach Fig. 8 hat jeder der (nicht einzeln bezeichneten) Leitungspole zwei Umkehrpunkte, die erfindungsgemäß jeweils durch Schaffung einer elektrischen Verbindung zwischen benachbart im Leitungskörper verlaufenden Einzelleitern geschaffen sind. Bei der Ausführung gemäß Fig. 9 haben drei der Elektrodenpole jeweils einen Umkehrpunkt im ihren Leitungsverlauf, während der vierte (zwischen dem Elektrodenanschluss 5.4 und der Elektrode 3.1) keinen Umkehrpunkt hat. Ähnlich ist die Verbindung zwischen Elektrodenanschlüssen und Elektroden bei der in Fig. 10 gezeigten Variante gestaltet; hier sind aber die Leiterabschnitte zwischen dem Umkehrpunkt und der jeweiligen Elektrode im distalen Abschnitt der Elektrodenleitung bei den drei mit einem Umkehrpunkt versehenen Leitungspolen gleich lang.

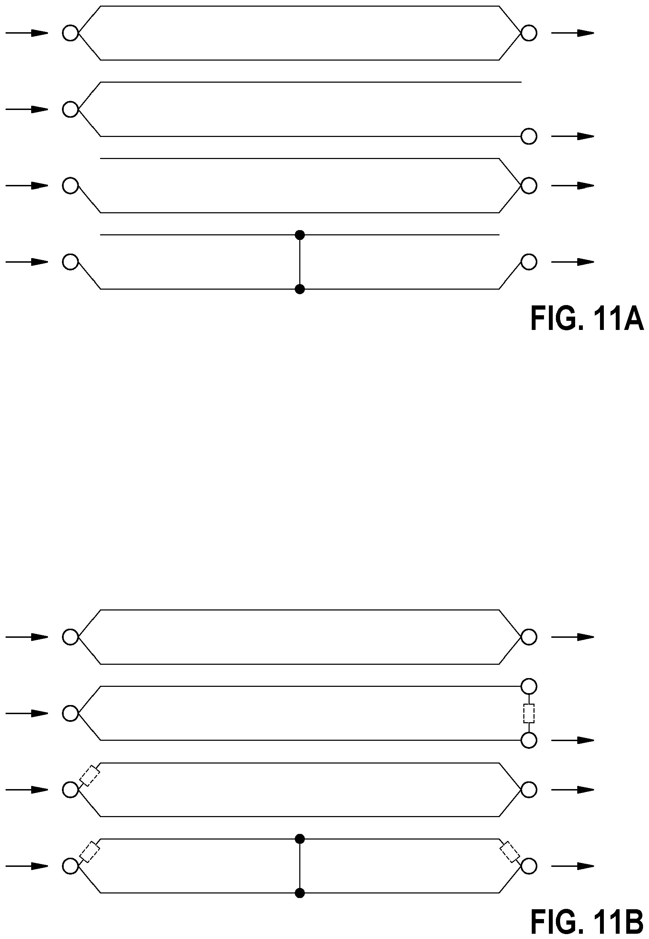

Die Fig. 11A und 11B zeigen schematisch Verbindungsvarianten mit einer zweipoligen Leitung, und zwar jeweils die Ausführungen einer Lecherleitung, die sich mit der erfindungsgemäßen Konstruktion leicht realisieren lassen und deren Effekt im MRT eine Dämpfung durch Fehlanpassung ist.

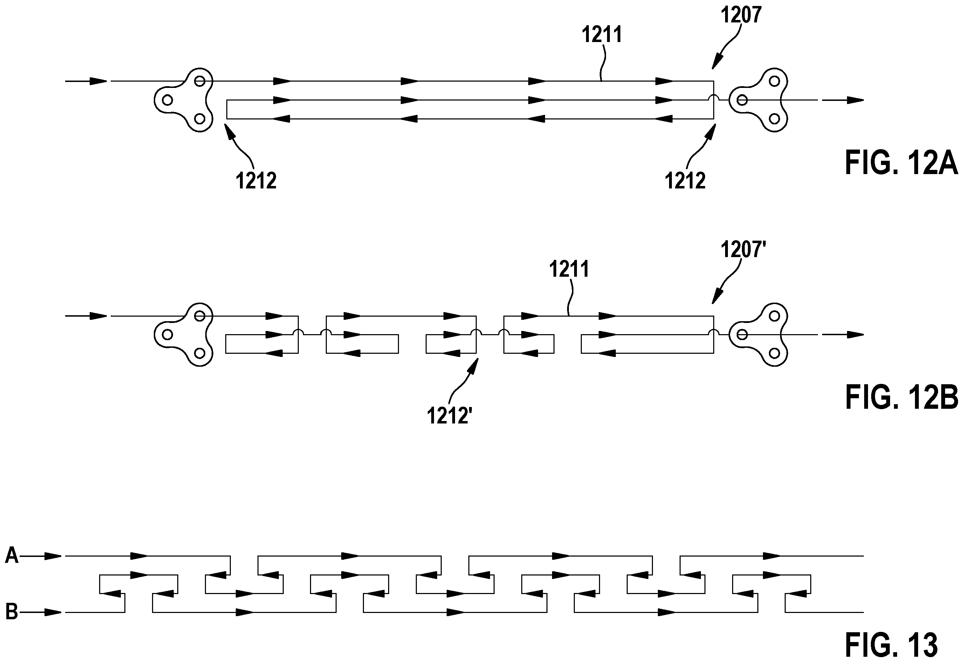

Fig. 12A und 12B zeigen zwei Varianten der Leitungsführung mit einem dreipoligen Leitungskomplex (einer Zuleitung) 1207. Hierbei sind jeweils alle drei separaten Leiter 1211 der Zuleitung 1207 bzw. 1207' in einen signaltragenden Leitungsverlauf einbezogen. Das Signal wird in einem Ende der Zuleitung in den in der Figur oben liegenden Einzelleiter eingespeist und am anderen Ende am mittleren Einzelleiter abgegriffen. Bei der Leitung 1207' gemäß Fig. 12A gibt es im Leitungsverlauf zwei Umkehrpunkte 1212, die nahe den Enden den Einzelleiter liegen, während bei der Ausführung nach Fig. 12B über die Längserstreckung der Leiter jeweils mehrere Verbindungs- und Umkehrpunkte 1212' geschaffen sind und die Stromflussrichtung mehrfach umgekehrt wird.

Die Zuleitung kann insgesamt die Funktion eines herkömmlichen Leiterseils übernehmen. In Ausgestaltungen beider Ausführungen können die Leitungslängen zwischen den Umkehrpunkten variiert und ggf. auch die Umkehrpunkte mit variierenden Abständen (somit zur Realisierung ungleich langer Leiterabschnitte mit unterschiedlicher Stromflussrichtung) festgelegt sein.

Fig. 13 zeigt schematisch einen Stromfluss, wie er in einer (als solche nicht gezeigten) vierpoligen Zuleitung realisiert werden kann, bei der die vier Leitungen zur Realisierung zweier elektrischer Verbindungen eingesetzt werden, wobei jeweils mehrere Umkehrpunkte vorgesehen sind. Die paarweise eingesetzten vier Leitungen können, z. B. für eine Zu- und eine Ableitung, getrennt verwendet oder auch parallel geschaltet werden. Eine spezielle Ausgestaltung kann jeweils in einer Verdrillung des Zuleitungskomplexes bestehen, und die Zuleitung kann außerdem in gewendelter Form in die Elektrodenleitung eingebaut sein.

Die Ausführung der Erfindung ist nicht auf die oben beschriebenen Beispiele und hervorgehobenen Aspekte beschränkt, sondern ebenso in einer Vielzahl von Abwandlungen möglich, die im Rahmen fachgemäßen Handelns liegen.

## Patentansprüche

1. Implantierbare medizinische Leitung zur Übertragung elektrischer Impulse an reizbares Körpergewebe und/oder von an Körpergewebe erfassten Signalen zu einer Erfassungs- und Auswertungseinheit, mit
einer distalen Elektrode oder einem distalen Sensor, oder Aktuator,
einem proximalen Elektroden- oder Sensor-/Aktuatoranschluss und
einem die Elektrode oder den Sensor oder Aktuator mit dem Elektroden- oder Sensor-/Aktuatoranschluss verbindenden, in einem Leitungskörper verlaufenden Leitungspol,
wobei der Leitungspol mindestens zwei separate und gegeneinander isolierte Leiter aufweist, die an mindestens einer Stelle miteinander elektrisch verbunden oder gekoppelt sind, welche als Übergabe- bzw. Umkehrpunkt im Leitungsverlauf vom proximalen Elektroden- oder Sensoranschluss zur distalen Elektrode oder dem distalen Sensor wirkt, **dadurch gekennzeichnet, dass** mindestens einer der separaten Leiter, nahe dem Umkehrpunkt, an einem Ende nicht angeschlossen ist.

2. Leitung nach Anspruch 1, wobei die separaten Leiter durch eine Löt-, Schweiß- oder Klebverbindung mittels leitfähigen Klebstoffs fest miteinander verbunden sind.

3. Leitung nach Anspruch 1, wobei die separaten Leiter durch Crimpen, insbesondere mittels Crimpschelle, fest miteinander verbunden sind.

4. Leitung nach einem der vorangehenden Ansprüche, wobei im Übergabe- bzw. Umkehrpunkt mindestens ein elektrisches Bauelement, insbesondere ein Widerstand, eine Kapazität, eine Induktivität, ein Halbleiterelement, ein Filterbauelement, eine elektronische Nachbildung dieser Elemente, ein Sensorbauelement oder eine Kombination von Bauelementen in einem Array oder einem Schaltkreis, vorgesehen ist.

5. Leitung nach einem der vorangehenden Ansprüche, wobei der Leitungspol n>2 separate und gegeneinander isolierte Leiter umfasst, von denen jeweils zwei derart an je einem Umkehrpunkt elektrisch miteinander verbunden sind, dass der Leitungspol einen bis (n -1) Umkehrpunkte aufweist.

6. Leitung nach einem der vorangehenden Ansprüche, mit mehreren Leitungspolen, die jeweils mindestens zwei separate und gegeneinander isolierte Leiter und mindestens einen Übergabe- bzw. Umkehrpunkt aufweisen.

7. Leitung nach einem der vorangehenden Ansprüche, wobei die separaten Leiter des oder eines Leitungspols parallel zueinander verlaufende gestreckte, insbesondere seilartig geflochtene, Leiter sind.

8. Leitung nach einem der vorangehenden Ansprüche, wobei die separaten Leiter des oder eines Leitungspols als, insbesondere ineinander gewickelte oder koaxial zu einer Längsachse der Leitung angeordnete, Leiterwendeln ausgebildet sind.

9. Leitung nach einem der vorangehenden Ansprüche, wobei jeweils einer der separaten Leiter des oder eines Elektrodenpols auf einem von Elektrodenpotentialen unabhängigen Potential liegt und insbesondere eine Abschirmwirkung hat.

10. Leitung nach einem der vorangehenden Ansprüche, wobei der oder ein Elektrodenpol entlang der Achse der Leitung parallel verlaufende oder wendelförmig um die Achse verlaufende separate und gegeneinander isolierte Leiter aufweist, und wobei ein oder zwei Übergabe- bzw. Umkehrpunkte gebildet sind.

11. Verfahren zur Herstellung einer implantierbaren medizinischen Leitung nach einem der vorangehenden Ansprüche, mit den Schritten:
Bereitstellung eines Paares oder einer Gruppe separater und gegeneinander isolierter Leiter in einer zur Herstellung einer implantierbaren medizinischen Leitung geeigneten Ausführung,
lokale Zerstörung der Isolierung der Leiter derart, dass ein unisolierter Leiterabschnitt jeweils zweier Leiter nahe beieinander liegt,
elektrisches Verbinden der beiden Leiter an den nahe beieinander liegenden unisolierten Leitungsabschnitten zur Bildung eines Übergabe- bzw. Umkehrpunktes im Leitungsverlauf von einem proximalen Elektroden- oder Sensoranschluss zu einer distalen Elektrode oder einem distalen Sensor, und Einbetten des auf vorgenannte Weise bearbeiteten Paares oder der Gruppe separater Leiter als Leitungspol in einen Leitungskörper und Anbringen einer Elektrode oder eines Sensors am distalen Ende und eines Elektroden- oder Sensoranschlusses am proximalen Ende des Elektrodenpols, **dadurch gekennzeichnet, dass** mindestens einer der separaten Leiter nahe dem Umkehrpunkt an einem Ende nicht angeschlossen wird.

12. Verfahren nach Anspruch 11, wobei das Zerstören der Isolierung und elektrische Verbinden der separaten Leiter zur Bildung des Übergabe- bzw. Umkehrpunktes durch Laserbearbeitung oder Widerstandsschweißen erfolgt.

13. Verfahren nach Anspruch 11, wobei das Zerstören der Isolierung und elektrische Verbinden der Leiter zur Bildung des Übergabe- bzw. Umkehrpunktes durch Crimpen, insbesondere unter Einsatz einer Crimpschelle, erfolgt.

14. Verfahren nach einem der Ansprüche 11-13, wobei die Bildung des Leitungskörpers durch Umspritzen des Elektrodenpols, insbesondere zusammenhängendes Umspritzen mehrerer Elektrodenpole, oder Extrudieren eines Kunststoffkörpers um den Elektrodenpol oder die Elektrodenpole ausgeführt wird.

## Claims

1. An implantable medical lead for transmitting electrical impulses to excitable bodily tissue and/or signals tapped at bodily tissue to a detection and evaluation unit, comprising
a distal electrode or a distal sensor, or actuator,
a proximal electrode connector or sensor/actuator connector, and
a lead pole which connects the electrode or the sensor or actuator to the electrode connector or sensor/actuator connector and which runs in a lead body,
wherein the lead pole comprises at least two separate conductors insulated with respect to one another which are electrically connected or coupled to one another at least at one point which functions as a transfer point or reversal point in the lead extension from the proximal electrode connector or sensor connector to the distal electrode or the distal sensor, **characterised in that**
at least one of the separate conductors close to the reversal point is not connected at one end.

2. The lead according to claim 1, wherein the separate conductors are securely interconnected by a soldered connection, welded connection, or bonded connection using conductive adhesive.

3. The lead according to claim 1, wherein the separate conductors are securely interconnected by crimping, in particular by means of a crimp clamp.

4. The lead according to any one of the preceding claims, wherein at least one electrical component, in particular a resistor, a capacitor, an inductor, a semiconductor element, a filter component, an electronic simulation of these elements, a sensor component, or a combination of components in an array or a circuit, is provided at the transfer point or reversal point.

5. The lead according to any one of the preceding claims, wherein the lead pole comprises n>2 separate conductors insulated with respect to one another, of which two are electrically interconnected at each reversal point such that the lead pole comprises one to (n-1) reversal points.

6. The lead according to any one of the preceding claims, comprising a plurality of lead poles, each of which comprises at least two separate conductors insulated with respect to one another and at least one transfer point or reversal point.

7. The lead according to any one of the preceding claims, wherein the separate conductors of the lead pole or of a lead pole are conductors that extend parallel to one another and in particular are woven in the manner of a rope.

8. The lead according to any one of the preceding claims, wherein the separate conductors of the lead pole or of a lead pole are designed as conductor helixes which in particular are interwoven or are disposed coaxially to a longitudinal axis of the lead.

9. The lead according to any one of the preceding claims, wherein one of the separate conductors of the electrode pole or of an electrode pole is at a potential that is independent of electrode potentials and in particular has a shielding effect.

10. The lead according to any one of the preceding claims, wherein the electrode pole or an electrode pole comprises separate conductors insulated with respect to one another which extend along the axis of the lead in parallel, or about the axis in the manner of a helix, and wherein one or two transfer or reversal points is/are formed.

11. A method for manufacturing an implantable medical lead according to any one of the preceding claims, comprising the steps of:
providing a pair or a group of separate conductors insulated with respect to one another in a form suitable for the manufacture of an implantable medical lead, locally destroying the insulation of the conductors such that an uninsulated conductor portion of one conductor is located close to an uninsulated conductor portion of a second conductor,
electrically connecting the two conductors at the uninsulated lead portions located close to one another to form a transfer point or reversal point in the lead extension from a proximal electrode connector or sensor connector to a distal electrode or a distal sensor; and
embedding the pair or group of separate conductors, which have been prepared as described above, as a lead pole in a lead body, and attaching an electrode or a sensor at the distal end and an electrode connector or sensor connector at the proximal end of the electrode pole,
**characterised in that** at least one of the separate conductors close to the reversal point is not connected at one end.

12. The method according to claim 11, wherein laser processing or resistance welding is used to destroy the insulation and electrically connect the separate conductors to form the transfer point or reversal point.

13. The method according to claim 11, wherein crimping, in particular using a crimp clamp, is used to destroy the insulation and electrically connect the conductors to form the transfer point or reversal point.

14. The method according to any one of claims 11-13, wherein the lead body is formed by encapsulating the electrode pole, in particular by coherently encapsulating a plurality of electrode poles, or by extruding a plastic body around the electrode pole or the electrode poles.

## Revendications

1. Ligne médicale implantable pour la transmission d'impulsions électriques à des tissus corporels excitables et/ou de signaux détectés sur des tissus corporels vers une unité de détection et d'exploitation, avec
une électrode distale ou un capteur distal, ou un actuateur,
une électrode proximale ou un connecteur de capteur/d'actuateur et
un pôle conducteur reliant l'électrode, ou le capteur, ou l'actuateur, avec le connecteur d'électrode ou de capteur/d'actuateur, s'étendant dans un corps conducteur,
où le pôle conducteur présente au moins deux conducteurs distincts et isolés l'un vis-à-vis de l'autre qui sont reliés ou couplés électriquement l'un à l'autre à au moins un endroit, lequel agit en tant que point de transmission, respectivement d'inversion, dans le tracé de la ligne à partir du connecteur d'électrode ou du capteur proximal jusqu'à l'électrode distale ou jusqu'au capteur distal, **caractérisée en ce qu'**au moins un des conducteurs distincts n'est pas raccordé à une extrémité près du point d'inversion.

2. Ligne selon la revendication 1, dans laquelle les conducteurs distincts sont reliés solidement ensemble par une liaison par brasage, par soudage ou par collage au moyen d'un adhésif conducteur.

3. Ligne selon la revendication 1, dans laquelle les conducteurs distincts sont reliés solidement ensemble par des sertissages, en particulier au moyen d'un collier de sertissage.

4. Ligne selon l'une des revendications précédentes, dans laquelle, au point de transmission, respectivement, d'inversion, au moins un composant électrique, en particulier, une résistance, un condensateur, un inducteur, un élément semiconducteur, un composant de filtrage, une simulation électronique de ces éléments, un composant de capteur ou une combinaison de composants dans un réseau ou un circuit, est prévu.

5. Ligne selon l'une des revendications précédentes, dans laquelle le pôle conducteur comprend n > 2 conducteurs distincts et isolés les uns vis-à-vis des autres, parmi lesquels, chaque fois, deux d'entre eux sont reliés ensemble électriquement à un point d'inversion de sorte que le pôle conducteur présente de un à (n - 1) points d'inversion.

6. Ligne selon l'une des revendications précédentes, avec plusieurs pôles conducteurs qui présentent respectivement au moins deux conducteurs distincts et isolés l'un vis-à-vis de l'autre et au moins un point de transmission, respectivement d'inversion.

7. Ligne selon l'une des revendications précédentes, dans laquelle les conducteurs distincts du ou d'un pôle conducteur sont des conducteurs allongés s'étendant parallèles les uns par rapport aux autres, en particulier tressés comme dans une corde.

8. Ligne selon l'une des revendications précédentes, dans laquelle les conducteurs distincts du ou d'un pôle conducteur sont conçus sous forme de bobinages conducteurs, en particulier enroulés à l'état enchevêtré ou disposés co-axialement par rapport à un axe longitudinal de la ligne.

9. Ligne selon l'une des revendications précédentes, dans laquelle, chaque fois, un des conducteurs distinct du ou d'un pôle conducteur se situe à un potentiel indépendant des potentiels d'électrodes et a en particulier un effet de blindage.

10. Ligne selon l'une des revendications précédentes, dans laquelle le ou un pôle d'électrode présente des conducteurs distincts et isolés les uns vis-à-vis des autres s'étendant parallèlement le long de l'axe de la ligne ou sous forme de bobines autour de l'axe, et où un ou deux points de transmission, respectivement d'inversion, sont formés.

11. Procédé de fabrication d'une ligne médicale implantable selon l'une des revendications précédentes, avec les étapes :
de mise à disposition d'une paire ou d'un groupe de conducteurs distincts et isolés les uns vis-à-vis des autres dans une configuration appropriée pour la fabrication d'une ligne médicale implantable,
de destruction locale de l'isolation des conducteurs de telle manière qu'un segment de conducteur non isolé se situe respectivement près de deux conducteurs proches, de liaison électrique des deux conducteurs sur les segments de conducteur non isolés situés l'un près de l'autre pour la formation d'un point de transmission, respectivement, d'inversion, dans le tracé de la ligne à partir d'un connecteur d'électrode ou de capteur proximal jusqu'à une électrode distale ou un capteur distal,
et
d'incorporation de la paire ou du groupe de conducteurs distincts façonné(e) de la manière précitée en tant que pôle conducteur dans un corps de ligne et de mise en place d'une électrode ou d'un capteur à l'extrémité distale et d'un connecteur d'électrode ou de capteur à l'extrémité proximale du pôle d'électrode,
**caractérisé en ce qu'**au moins un des conducteurs distincts n'est pas raccordé à une extrémité près d'un point d'inversion.

12. Procédé selon la revendication 11, dans lequel la destruction de l'isolation et la liaison électrique des conducteurs distincts, pour la formation du point de transmission, respectivement, d'inversion, est réalisée par un traitement au laser ou un soudage par résistance.

13. Procédé selon la revendication 11, dans lequel la destruction de l'isolation et la liaison électrique des conducteurs pour la formation du point de transmission, respectivement, d'inversion, est réalisée par un sertissage, en particulier, moyennant l'emploi d'un collier de sertissage.

14. Procédé selon l'une des revendications 11 à 13, dans lequel la formation du corps de ligne est effectuée par un surmoulage par pulvérisation du pôle d'électrode, en particulier par le surmoulage par pulvérisation à l'état suspendu de plusieurs pôles d'électrodes, ou par l'extrusion d'un corps en matière plastique autour du pôle d'électrode ou des pôles d'électrodes.
